# EUROPEAN PATENT APPLICATION

(11) **EP 4 332 235 A1**
(43) Date of publication of application: **06.03.2024**
(21) Application number: 23193556.0
(22) Date of filing: 25.08.2023
(51) Int. Cl.: C12Q 1/6806, C12Q 1/6827

(54) **HIGHLY SENSITIVE METHODS FOR ACCURATE PARALLEL QUANTIFICATION OF VARIANT NUCLEIC ACIDS**

(30) Priority: 31.08.2022 US 202217900109
(71) Applicant: Genomill Health Oy, 20520 Turku (FI)
(72) Inventor: Pursiheimo, Juha-Pekka, 20520 Turku (FI); Hirvonen, Tatu, 20520 Turku (FI); Korkiakoski, Anttoni, 20520 Turku (FI); Tamminen, Manu, 20520 Turku (FI)
(74) Representative: Moosedog Oy

(57) **Abstract**

The present invention disclosure relates to a next generation DNA sequencing method and use for accurate and massively parallel quantification of one or more nucleic acid targets, for example in large volumes of unpurified sample material. More particularly, the invention is related to a method and a kit comprising probes for detecting and quantifying genetic targets in complex samples. The invention includes at least target-specific nucleic acid probes per genetic target (first probe, second probe and target-specific probe) and a bridge oligo or bridge oligo complex.

## Description

### TECHNICAL FIELD

The present invention disclosure relates to improved next generation DNA sequencing methods for accurate and massively parallel quantification of one or more nucleic acid targets. More particularly, the disclosure is related to methods and kits comprising probes for detecting and quantifying genetic targets in complex DNA pools primarily used for genetic target and variant detection.

### BACKGROUND

With the advancement in the technology to study genetic variation, detection of the same in plants and animals is not cumbersome. However, detecting and accurately quantifying genetic variations such as mutations, in particular in samples having weak signals is currently still cumbersome, laborious and expensive, despite decreased sequencing costs. Various problems can be expressed more accurately such as specificity in order to detect genetic signals against a consensus background, sensitivity in order to detect weak genetic signals, accuracy for accurate quantification of the detected signals, throughput number of targeted genetic targets per assay, cost per assay, scaling to determine the assay cost scale when assaying multiple samples in parallel and turn-over to determine how long is the time from sampling to the results.

Currently, the typical quantification methods for liquid biopsies and conceptually similar assays (such as antibiotic resistance gene detection) include quantitative PCR (qPCR), array qPCR, digital PCR, multiplex ligation-dependent probe Amplification (MLPA) or quantification from next-generation DNA sequencing data. While the quantification methods are robust and well-established methods, each of the method is associated with specific problems discussed in closer detail below:
Quantitative PCR: Quantitative PCR (qPCR), is a technique which includes the amplification of a targeted DNA molecule during the PCR, i.e. in real-time. Real-time PCR can be used quantitatively (quantitative real-time PCR), and semi-quantitatively, i.e. above/below a certain amount of DNA molecules (semi quantitative real-time PCR). Quantitative PCR (qPCR) is a gold standard of genetic target quantification. Currently, the laboratory cost of a qPCR reaction is approximately $2. However, counting in the considerable hands-on time (labour cost) for setting up the reaction, the need for standard curves, along with replicates for each quantified target, the real cost is in fact much higher. The amount of hands-on time scales steeply with an increasing number of samples since a separate quantification experiment is required for each genetic target.

Array PCR: PCR Arrays are the most reliable tools for analyzing the expression of a relevant pathway- or disease-focused panel of genes. Each 96-well plate, 384-well plate, or 100-well disc PCR Array includes SYBR Green-optimized primer assays for a thoroughly researched panel of focused panel of genes. A newer iteration of the qPCR technology is array qPCR which miniaturizes the individual qPCR reactions. Array PCR brings down the cost of an individual qPCR reaction and improves the scalability of the method to multiple targets and samples. However, the method is currently limited to profiling 384 targets from 12 samples (or conversely 12 targets from 384 samples) at a cost of thousands of dollars per chip plus a large capital cost of the read-out infrastructure. Profiling thousands of samples using the aforementioned setup, therefore, remains prohibitively expensive.

Digital PCR: Digital polymerase chain reaction (digital PCR, DigitalPCR, dPCR, or dePCR) is a method to provide absolute quantification of targets through droplet-microfluidics and fluorescent detection. The methodology is relatively cost-effective (one target per sample costs around $3), but the hands-on time for preparing, setting-up and running individual experiments for each target in each sample scales poorly to thousands of samples.

Multiplex Ligation-dependent Probe Amplification (MLPA) provides an approach to simplify the detection of multiple genetic targets in individual samples. However, MLPA provides only relative quantification of targets, and requires a separate detection experiment for each sample. More recently, a variant of MLPA introduces concepts from DNA barcoding. The concept permits a better quantitative resolution and sample multiplexing than the traditional MLPA workflow.

Next generation sequencing-based approaches: Next-generation sequencing (NGS), also known as high-throughput sequencing that makes sequence-based gene expression analysis a "digital" alternative to analog techniques. Target counting from next-generation DNA sequencing data is becoming increasingly attractive as the cost of DNA sequencing keeps decreasing, and is currently used for instance in noninvasive prenatal testing screening. However, the current approach suffers from high sequencing library preparation costs and sequencing efforts that is wasted on sequencing non-relevant genetic targets. For instance, in cancer-related liquid biopsies, non-targeted approaches result in wastage of sequencing effort on oncologically non-relevant loci. In fetal diagnostics, non-targeted sampling of loci considerably limits the statistical options for interpreting the data. Guardant Health Inc provides more targeted sequencing approach, where an array of RNA capture probes enriches targets for next-generation DNA sequencing.

Akhras et al. (2007) PLoS ONE 2(2):e223 disclose a multiplex pathogen detection assay involving barcoded target-specific probes, target circularization and sequencing. Use of a bridging oligonucleotide to ligate the target-specific probes is also disclosed.

WO2018109206 describes methods for the detection of analytes in a sample using padlock probes and rolling circle amplification. The use of a bridging oligo is not described.

WO2019038372 (incorporated by reference) describes a next-generation sequencing approach wherein target sequences of interest are selectively amplified by in vitro transcription from ligation complexes containing a promoter for T7 polymerase, followed by cDNA synthesis and sequencing. While this method allows accurate and parallel detection and quantification of many target sequences in a sample, more complex, large volume, dilute and/or impure samples remain challenging.

Therefore, in light of the foregoing discussion, there exists a need to overcome the aforementioned drawbacks such as, but not limited to, specificity, sensitivity, accuracy, throughput, cost, scaling and turn-over through an accurate and massively parallel quantification of nucleic acid targets.

### SUMMARY OF THE INVENTION

The present invention provides a method for using next-generation sequencing for highly sensitive, scalable and accurate target quantification from large volume samples (up to tens of milliliters) and/or dilute and/or non-purified sample material. Furthermore, an RNA amplification step such as described in WO2019038372 is avoided, rendering the method more simple. Moreover, the method of the invention comprises an target sequence amplification step wherein an additional target-specific probe, or multiple additional target-specific probes, is/are used to specifically amplify, and thus specifically enrich for, particular sequences, such as rare sequences. This allows the detection of such rare sequences in a sample containing a surplus of related but non-identical sequences. For example, the method can be used to detect a rare allele of a gene in samples containing a surplus of other alleles.

In a first main aspect, the invention relates to a method for the detection of one or more target nucleotide sequence in a sample, the method comprising the steps of:
(i) providing for each target nucleotide sequence in the sample:
   a first probe, a second probe and a bridge oligo or a plurality of oligonucleotides capable of annealing to each other to form a bridge oligo complex,
   wherein the first probe comprises, starting from the 5' end of the molecule, a first bridge oligo-specific sequence, optionally a first sequence barcode, and a first target specific portion at the 3' end of first probe;
   wherein the second probe comprises, starting from the 5' end of the molecule, a second target specific portion, optionally a second sequence barcode, and a second bridge oligo-specific sequence at the 3' end of second probe;
   wherein the bridge oligo or bridge oligo complex contains sequences complementary to the first bridge oligo-specific sequence and the second bridge oligo-specific sequence in the first probe and the second probe, respectively, and optionally a third barcode;
   wherein at least one of the first sequence barcode or the second sequence barcode or the third barcode is present in the first probe or the second probe or the bridge oligo or bridge oligo complex, respectively;
   and wherein optionally at least one of the first probe or the second probe or the bridge oligo or bridge oligo complex comprises a recognition sequence for an endonuclease;
(ii) contacting, for each of the one or more target nucleotide sequence, the first probe and the second probe with the bridge oligo or plurality of oligonucleotides capable of annealing to each other to form a bridge oligo complex and allow self-annealing into a plurality of ligation complexes;
(iii) contacting nucleic acids present in the sample to be tested for the target nucleotide sequences with the ligation complexes;
(iv) allowing the first target specific portion and the second target specific portion of the respective first probe and the second probe to hybridize to essentially adjacent sections on the target sequence, thereby forming a hybridization complex;
(v) ligating the probes in the hybridization complexes to provide ligated ligation complexes;
(vi) allowing the ligated ligation complexes to dissociate from the target nucleotide sequence;
(vii) adding a target-specific probe comprising a sequence corresponding to the target nucleotide sequence, wherein said target-specific probe is capable of annealing with the ligated ligation complexes, and allowing the target-specific probe to anneal to the ligated ligation complexes thereby forming amplification templates;
(viii) amplifying nucleic acids from the amplification templates using rolling circle amplification with a strand-displacing polymerase thereby obtaining single-stranded concatemeric sequences;
(ix) optionally, provided a recognition sequence as specified in step (i) is present, performing a step to obtain nucleic acid fragments by:
   (a) cleaving the single-stranded concatemeric sequences obtained in step (viii), or
   (b) subjecting the amplified one or more single-stranded concatemeric sequence obtained in step (viii) to annealing with a specific oligonucleotide containing a recognition sequence for an endonuclease wherein the oligonucleotide anneals with the recognition sequence specified in step (i) such that a recognition site for the endonuclease is obtained and cleaving the annealed complexes with said endonuclease;
(x) subjecting the concatemeric sequence obtained in step (viii) or the nucleic acid fragments obtained in step (ix) to sequencing technology to determine the barcode sequence(s); and
(xi) identifying the presence and/or number of the target nucleotide sequence in the sample by determination of at least part of the first target specific portion and/or the second target specific portion, and/or at least part of the first barcode and/or the second barcode, and/or at least part of the third barcode.

### DESCRIPTION OF THE FIGURES

FIG. 1 illustrates a flow diagram of the Multiplexed Ligation Assay (MLA) according to an embodiment of the invention.
FIG. 2A, 2B, 2C and 2D illustrate principle combinations of probes according to embodiments of the invention.
FIG. 3 illustrates the use of the target-specific probe according to an embodiment of the invention, here to detect a rare mutation.
FIG 4 illustrates two boosted sequencing libraries with two different target concentrations.
FIG 5 illustrates the effect of boosting on a panel of 12 gene fusion targets.

### DETAILED DESCRIPTION OF THE INVENTION

### Definitions:

Target Nucleotide Sequence: The term target nucleotide sequence may be any nucleotide sequence of interest of which its detection is required. It will be understood that the term given refers to a sequence of contiguous nucleotides as well as to nucleic acid molecules with the complementary sequence. The target sequence in some embodiments is a nucleotide sequence that represents or is associated with a polymorphism.

Polymorphism: The term polymorphism refers to an occurrence of two or more genetically determined alternative sequences or alleles in a population. A polymorphic marker or site is the locus at which sequence divergence occurs. A polymorphic locus may be as small as one base pair. Samples: The term samples is used herein for two or more samples which contain two or more target sequences. Samples as provided in a method according to the invention may have been prepared in order to extract at least the target nucleic acids and make those accessible to the probes as used in the invention. In particular, in some embodiments, the samples each comprise at least two different target sequences, preferably at least 100, more preferably at least 250, more preferably at least 500, most preferably at least 2000, or more. The term samples may refer to but is not limited to two or more samples obtained from a human/animal body, including urine, biopsies, saliva and other secretions, exhaled moisture extracts, tissue, blood plasma (liquid biopsies), or two or more samples obtained from the environment, including water, wastewater, soil, plants, or two or more samples containing viruses or bacteria or the like. In one embodiment, the plurality of samples includes a blood sample, a saliva sample, a urine sample or a feces sample, a sample of another body fluid or an extract from body material, for example hair or skin flakes.

Probe: The term probe is a fragment of DNA or RNA of variable length (usually 50-1000 bases long, preferably 50 - 200 bases long) which can be used in DNA or RNA samples to detect the presence of nucleotide sequences (the DNA or RNA target) that are complementary to the sequence in the probe. The sections of the oligonucleotide probes that are complementary to the target sequence are designed such that for each target sequence in a sample, a pair of a first and a second probe is provided, whereby the probes each contain a section at their extreme end that is complementary to a part of the target sequence. Furthermore, the present disclosure describes a bridge oligo or bridge oligo complex that is used for joining the first probe and the second probe. In addition, an additional target-specific probe, or boosting probe, is used which comprises a sequence that corresponds to part of the target sequence. Universal: The term universal when used to describe an amplification procedure refers to a sequence that enables the use of a single primer or set of primers for a plurality of amplification reactions. The use of such primers greatly simplifies multiplexing in that only two primers are needed to amplify a plurality of selected nucleic acid sequences. The term universal when used to describe a priming site is a site to which a universal primer will hybridize. It should also be noted that "sets" of universal priming sequences/primers may be used.

Hybridization: The term hybridization (or hybridisation) describes the process of DNA or RNA molecules annealing to complementary DNA or RNA. DNA or RNA replication and transcription of DNA into RNA both rely on nucleotide hybridization.

Ligation: The term ligation is the joining of two nucleic acid fragments through the action of an enzyme. DNA ligases are enzymes capable of catalyzing the formation of a phosphodiester bond between (the ends of) two polynucleotide strands bound at adjacent sites on a complementary strand. In one embodiment, ligation can also be performed chemically, in particular if both adjacent ends of the polynucleotides are modified to allow chemical ligation.

Amplification: The term amplification as used herein denotes the use of a DNA polymerase to increase the concentration of a particular nucleotide sequence within a mixture of nucleotide sequences. "PCR" or "Polymerase Chain Reaction" is a rapid procedure for in vitro enzymatic amplification of a specific DNA/RNA segment. The DNA/RNA to be amplified may be denatured by heating the sample. The term primer is a strand of RNA or DNA (generally about 18-22 bases) that serves as a starting point for DNA synthesis. It is required for DNA replication because the enzymes that catalyze this process, DNA polymerases, can only add new nucleotides to an existing strand of DNA.

Polymerase: A polymerase is an enzyme that synthesizes long chains or polymers of nucleic acids. DNA polymerase and RNA polymerase are used to assemble DNA and RNA molecules, respectively, by copying a DNA or RNA template strand using base-pairing interactions.

High throughput: The term high throughput denotes the ability to simultaneously process and screen a large number of DNA samples; as well as to simultaneously screen large numbers of different genetic loci within a single DNA sample. High-throughput sequencing or screening, often abbreviated as HTS is a method for scientific experimentation especially relevant to effectively screen large amounts of samples simultaneously.

Endonuclease: An endonuclease is an enzyme that cleaves DNA double or single strand at a random or specified location.

Barcode: Probes and oligos used in the present invention may comprise one or more barcodes consisting of nucleotide sequences. Barcode sequences may comprise target nucleotide sequence identifier sequences, sample identifier sequences and/or molecular barcodes (also termed Unique Molecular Identifiers) for target enumeration. Barcode sequences may comprise random sequences.

As described above, the disclosure relates to a method for the high-throughput detection of target nucleotide sequence detection in a very large number of samples by leveraging ligation-dependent assays. The disclosure provides a method for determining the sequences of genetic targets in complex nucleic acid pools using techniques permitted by next generation sequencing. The disclosure also provides a method to profile multiple genetic targets in a number of samples, preferably a very large number of samples, by leveraging ligation-dependent assays. The disclosure provides a method for the multiplex ligation-dependent probe amplification enabling querying different target nucleic acids in a plurality of samples. The methods of the present invention allow the sequencing of the one or more target nucleotide sequence in a plurality of samples providing a plurality of different probe sets for different target nucleic acids. Unique sequence identifiers are used for the identification of the genetic targets and absolute quantification of individual samples from the sample pool when processing the sequencing data.

In a first main aspect, the invention relates to a method for the detection of one or more target nucleotide sequence in a sample, the method comprising the steps of:
(i) providing for each target nucleotide sequence in the sample:
   a first probe, a second probe and a bridge oligo or a plurality of oligonucleotides capable of annealing to each other to form a bridge oligo complex,
   wherein the first probe comprises, starting from the 5' end of the molecule, a first bridge oligo-specific sequence, optionally a first sequence barcode, and a first target specific portion at the 3' end of first probe;
   wherein the second probe comprises, starting from the 5' end of the molecule, a second target specific portion, optionally a second sequence barcode, and a second bridge oligo-specific sequence at the 3' end of second probe;
   wherein the bridge oligo or bridge oligo complex contains sequences complementary to the first bridge oligo-specific sequence and the second bridge oligo-specific sequence in the first probe and the second probe, respectively, and optionally a third barcode;
   wherein at least one of the first sequence barcode or the second sequence barcode or the third barcode is present in the first probe or the second probe or the bridge oligo or bridge oligo complex, respectively;
   and wherein optionally at least one of the first probe or the second probe or the bridge oligo or bridge oligo complex comprises a recognition sequence for an endonuclease;
(ii) contacting, for each of the one or more target nucleotide sequence, the first probe and the second probe with the bridge oligo or plurality of oligonucleotides capable of annealing to each other to form a bridge oligo complex and allow self-annealing into a plurality of ligation complexes;
(iii) contacting nucleic acids present in the sample to be tested for the target nucleotide sequences with the ligation complexes;
(iv) allowing the first target specific portion and the second target specific portion of the respective first probe and the second probe to hybridize to essentially adjacent sections on the target sequence, thereby forming a hybridization complex;
(v) ligating the probes in the hybridization complexes to provide ligated ligation complexes;
(vi) allowing the ligated ligation complexes to dissociate from the target nucleotide sequence;
(vii) adding a target-specific probe comprising a sequence corresponding to the target nucleotide sequence, wherein said target-specific probe is capable of annealing with the ligated ligation complexes, and allowing the target-specific probe to anneal to the ligated ligation complexes thereby forming amplification templates;
(viii) amplifying nucleic acids from the amplification templates using rolling circle amplification with a strand-displacing polymerase thereby obtaining single-stranded concatemeric sequences;
(ix) optionally, provided a recognition sequence as specified in step (i) is present, performing a step to obtain nucleic acid fragments by:
   (a) cleaving the single-stranded concatemeric sequences obtained in step (viii), or
   (b) subjecting the amplified one or more single-stranded concatemeric sequence obtained in step (viii) to annealing with a specific oligonucleotide containing a recognition sequence for an endonuclease wherein the oligonucleotide anneals with the recognition sequence specified in step (i) such that a recognition site for the endonuclease is obtained and cleaving the annealed complexes with said endonuclease;;
(x) subjecting the concatemeric sequence obtained in step (viii) or the nucleic acid fragments obtained in step (ix) to sequencing technology to determine the barcode sequence(s); and
(xi) identifying the presence and/or number of the target nucleotide sequence in the sample by determination of at least part of the first target specific portion and/or the second target specific portion, and/or at least part of the first barcode and/or the second barcode, and/or at least part of the third barcode.

In one embodiment, the method is for the high-throughput detection of one or more target nucleotide sequence in a plurality of samples, wherein a plurality of samples is provided and wherein, preferably, step (ii) is performed for each of the samples in a separate tube. In one embodiment, the plurality of the samples is pooled prior to step (viii).

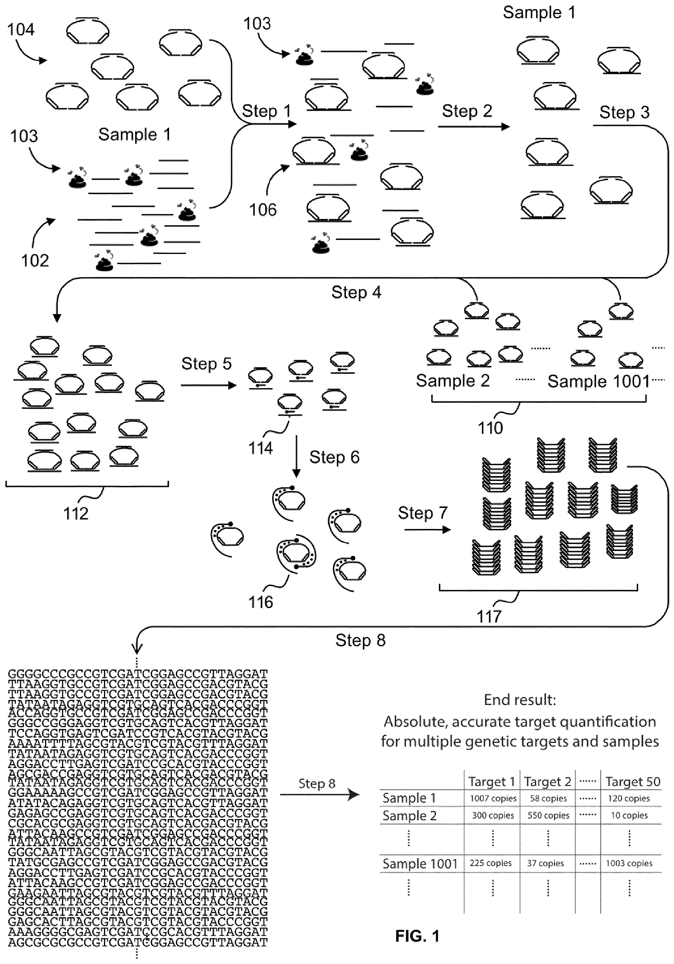

Figure 1 provides a non-limiting illustration of an embodiment of the method of the invention.

The methods of the present invention utilize four or more nucleic acid molecules, out of which three target-specific nucleic acid probes (first probe, second probe and target-specific probe) are specific for a genetic target and one or more nucleic acid probe typically is universal (bridge oligo or bridge oligo complex). The first probe and second probe hybridize to the bridge probe or bridge oligo complex, forming a ligation complex. The ligation complexes (containing one or more barcode sequences) having target identification sites on the sample DNA or RNA are allowed to hybridize against complementary target sequences of the query sample. After hybridization, the first and second probe are ligated chemically or enzymatically by a DNA ligase to form ligated ligation complex. In the present invention, a plurality of such ligated ligation complexes will form during the sample analysis in the plurality of samples to be analyzed.

A "plurality of samples" may refer to, but is not limited to, two or more samples obtained from the human or animal body, including biopsies, saliva and other secretions, exhaled moisture extracts, tissue, blood plasma (liquid biopsies), two or more samples obtained from environment, including water, wastewater, soil, plants, or two or more samples containing viruses or bacteria or the like. In one embodiment, the sample is used without any prior purification or concentration of nucleic acid. In another embodiment, the sample may be pre-treated, for instance lysing cells to expose nucleic acid.

The target sequence may include any nucleotide sequence of interest against which the detection is required. The target nucleotide sequence of the disclosure may be obtained from, but not limited to, a fraction of DNA in the patient's blood or a fraction of DNA in maternal blood. A fraction of the DNA in the patient's blood may for example be obtained from apoptotic/necrotic cancer cells or a fraction of DNA in maternal blood from fetal and/or maternal origin. Further, the results of analysis are used to, for instance, assess the risk of an individual to a given type of cancer, to determine the efficacy of a given treatment against a given cancer, the development of a drug-resistance-related mutations in a tumor, or the risk of a fetus carrying genetic disorders such as common trisomies Down, Patau and Edwards syndromes. In certain embodiments, the method comprises providing, for each target nucleotide sequence, a plurality of different probe sets.

As used herein, the term probe sets includes a first probe, a second probe and one or more bridge oligo.

In certain embodiments, the first probe includes, starting from the 5' end of the molecule, optionally a 5' phosphate, a first bridge oligo-specific sequence, optionally a first universal sequence, optionally a first sequence barcode, and a first target specific portion at its 3' end.

In certain embodiments, the second probe includes, starting from 5' end of the molecule, optionally a 5' phosphate, a second target specific portion, optionally a second sequence barcode, optionally a second universal sequence, and a second bridge oligo-specific sequence at its 3' end.

In some embodiments, either the first probe or the second probe contains at least one of the first sequence barcode or the second sequence barcode. The first sequence barcode or the second sequence barcode, or both, may be random sequences or may contain target nucleotide sequence identifier sequences, sample identifier sequences and/or molecular barcodes for target enumeration.

The bridge oligo or plurality of bridge oligos contains sequences complementary to the first and second bridge oligo-specific sequences in the first and second probe, respectively, optionally a universal sequence, and/or or may contain a third barcode which may be a random sequence or may contain a sample or sequence identifier sequence. In this respect, third barcode does not necessarily mean that there are already a first and a second barcode present. As described earlier, at least one barcode should be present in the ligated ligation complex, that enables to uniquely define the complex within all ligation complexes in all samples tested.

Optionally, at least one of: the first probe, the second probe, the one or more bridge oligo or the target-specific probe comprises a recognition sequence for an endonuclease. An endonuclease recognition sequence enables the cleavage of the concatemeric sequence. In one embodiment, the recognition sequence is a recognition sequence for a restriction endonuclease such as EcoRI. In another embodiment, the recognition sequence is a recognition sequence for a homing endonuclease such as I-CeuI. In another embodiment the recognition sequence is a recognition sequence for a guided DNAaseI or CRISPR-Cas-like cleavage system. In another embodiment, the recognition sequence is a recognition sequence for a nicking endonuclease.

Optionally, at least one of the first probe or the second probe or the one or more bridge oligo comprises a first capture moiety. A first capture moiety, when used herein, refers to a moiety, such as a chemical group, which allows the probe, ligation complex or hybridization complex to be captured by, i.e. bound to, a second capture moiety which is linked to a solid support. Any suitable capture moiety known in the art may be used for this purpose. A well-known suitable example is the capture of biotinylated molecules using streptavidin-coated magnetic beads. Thus, in one embodiment, the first capture moiety is a biotin moiety, which can interact with a streptavidin or avidin moiety (the second capture moiety) linked to a solid support, such as a magnetic bead. Other options include biotin derivatives such as dual-biotin, desthiobiotin or photocleavable biotin which can be used for conjugation with streptavidin/avidin. Further options include the use of thiol and acrydite groups for acrydite/acrylamide conjugation, alkyne and azide groups for click chemistry and digoxigenin for anti-digoxigenin antibody conjugation. The conjugation partners can be provided on any solid surfaces such as beads (magnetic or otherwise) or solid supports. Accordingly, in one embodiment of the method of the invention, at least one of: the first probe, the second probe, the barcode loop oligo or the one or more bridge oligo comprises a first capture moiety, and between steps (iv) and (v) an intermediate step (iv)(a) is performed which comprises bringing the hybridization complex in contact with a solid support comprising a second capture moiety, allowing the first capture moiety and the second capture moiety to interact such that the hybridization complexes become linked to the solid support and separating the solid-support-linked hybridization complexes from components of the samples that are not linked to the solid-support.

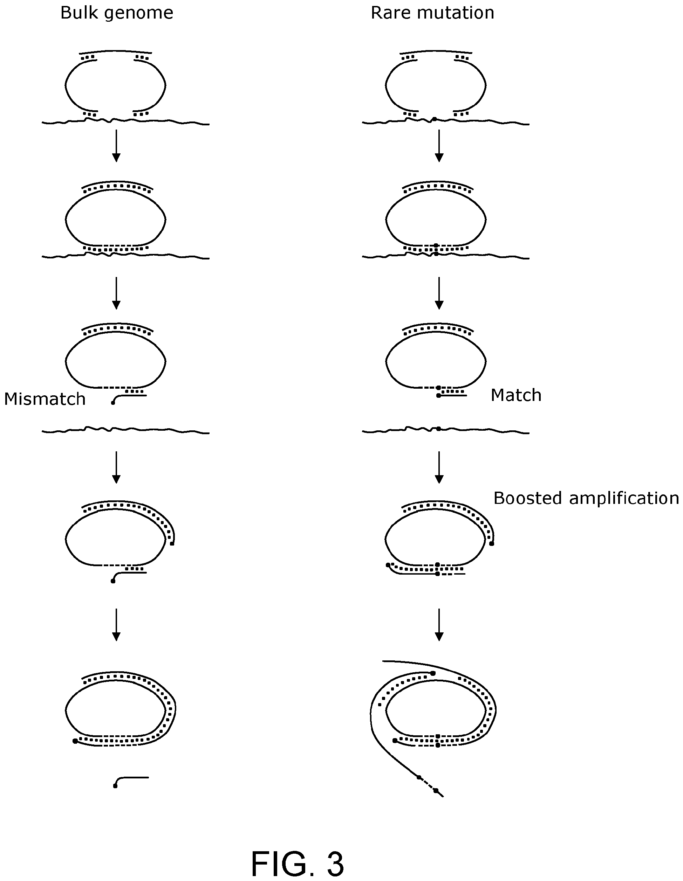

The first target specific portion, the second target specific portion, the first bridge oligo-specific sequences, and/or the second bridge oligo-specific sequences, preferably contain independently from one another at least one chemically modified nucleotide to increase probe binding. The chemical modifications that increase probe binding include, but are not limited to, ribonucleic acids, peptide nucleic acids, and locked nucleic acids (e.g. as illustrated in Figure 3 of WO2019038372, incorporated herein by reference). In one embodiment, the bridging portion of the first probe or the second probe, or both, comprise(s) chemically modified bases to permit improved binding to the bridge oligo or bridge oligo complex. In another embodiment, the first target specific portion, the second target specific portion, the first bridge oligo-specific sequences, and/or the second bridge oligo-specific sequences, contain independently from one another, one or more chemically modified nucleotides. In certain embodiments, chemical modifications permit chemical ligation of adjacent probes.

The aforementioned probes bind to adjacent genetic loci, i.e. adjacent sections of the target nucleotide sequence. However, the sections are not completely adjacent, but are at least 15 base pairs, such as at least 20, at least 25 or at least 30 base pairs apart. In preferred embodiments, the adjacent sections are not more than 500 base pairs apart, for example not more than 200 base pairs apart, such as not more than 100 or not more than 50 base pairs apart.

In some embodiments, the first probe, the second probe, the one or more bridge oligo or the target-specific probe may include adapter sequences for a DNA sequencing platform such as (but not limited to) Illumina MiSeq, NextSeq or NovaSeq. These adapter sequences permit the resulting sequencing libraries to bind to the detection parts of the sequencing devices such as Illumina flow cells.

Furthermore, in some embodiments, the bridge oligo or plurality of bridge oligonucleotides forming bridge oligo complex comprises:
(i) one to five 3' protruding bases (i.e. additional bases which do not form double helix with the second probe), and/or
(ii) 3' phosphate, and/or
(iii) one or more phosphorothioate modifications within three positions from the 3' end.

In one embodiment of the method of the invention, the method comprises the use of a plurality of oligonucleotides capable of annealing to each other to form a bridge oligo complex and said plurality of oligonucleotides comprises a barcode loop oligo,
wherein the barcode loop oligo comprises, starting from the 5' end of the molecule, a third bridge oligo-specific sequence, a barcoded loop sequence and a fourth bridge oligo-specific sequence, and
wherein the one or more other bridge oligo comprises sequences complementary to the third bridge oligo-specific sequence and the fourth bridge oligo-specific sequence in the barcode loop oligo.

Thus, the barcode loop oligo comprises a loop section flanked by two sections that can hybridize with the one or more bridge oligo to form a bridge oligo complex. The loop section does not hybridize with the one or more bridge oligo or bridge oligo complex and comprises a barcode. In one embodiment, the barcoded loop sequence comprises the third barcode.

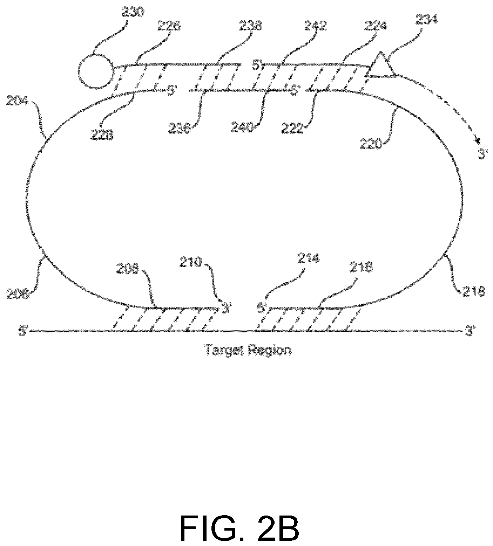

Before contacting the probes with the sample comprising the target sequences, the first probe and the second probe are brought in contact with the bridge oligo or plurality of oligonucleotides capable of forming a bridge oligo complex, preferably for each of the samples in a separate tube, and self-annealing into ligation complexes is allowed (step (ii)). In an embodiment wherein the bridge is not one oligo, but a plurality of oligonucleotides, such as three or five oligonucleotides, capable of annealing to each other to form a bridge oligo complex (illustrated herein in Figure 2B), the plurality of oligonucleotides may be pre-annealed before annealing with the first and second probes or all annealing steps may be done at once.

Preferably each ligation complex is unique for the combination of the first target specific sequence, the second target specific sequence and one or more barcode sequences. This enables enumeration of the target sequences after amplification and analysis of the results.

Thereafter, one or more target nucleotide sequences in the plurality of samples is brought into contact with the plurality of ligation complexes (step (iii)). The first target specific portion and the second target specific portion of the respective first probe and the second probe hybridize to essentially adjacent sections on the target sequence, thereby forming a hybridization complex (step (iv)). As mentioned above, the adjacent sections on the target sequence are typically between 15 and 500 base pairs apart.

In some embodiments, the sample has a volume of more than 100 microliters, e.g. more than 1 ml. In a further embodiment, the sample has a nucleic acid concentration below 5 pmol, such as below 1 pmol, for example below 200 fmol. In one embodiment, the plurality of samples includes one or more blood samples, one or more saliva samples, one or more urine samples or one or more feces samples.

Subsequently, in some embodiments, if at least one of the first probe or the second probe or the bridge oligo or the oligonucleotides forming the bridge oligo complex comprises a first capture moiety, the hybridization complex(es) are brought in contact with a solid support comprising a second capture moiety and the first capture moiety and the second capture moiety are allowed to interact such that the hybridization complex(es) become linked to the solid support (optional step (iv)(a)). Thereafter, the solid-support-linked hybridization complexes are separated from components of the samples that are not linked to the solid-support. If the solid supports are magnetic beads, the beads may be immobilized using a magnet and the remaining liquid sample may be removed. Optionally, a wash step is performed before proceeding.

Step (iv)(a) results in a purification and enrichment for nucleic acid, allowing improved results in particular for highly impure samples. In one embodiment, the method of the invention does not comprise a step of enriching for nucleic acids prior to step (iv)(a). Thus, in one embodiment, the method does not contain prior to step (v) a step wherein nucleic acids in the original sample are concentrated more than 2-fold, more than 10-fold, or more than 100-fold. In another embodiment, the method of the invention does not include a purification step subsequent to the ligation in step (vi).

Subsequently, ligation of the probes in the formed hybridized complexes is carried out either enzymatically or chemically to provide ligated ligation complexes (step (v)). Optionally as a part of step (v), a gap between the first probe and the second probe, if present, may be filled by introducing a polymerase and one or more nucleotides. The polymerase adds nucleotides (a) complimentary to the bridge oligo sequence and/or (b) complimentary to the barcode sequence and thereby fills in the two gaps between the first probe and the second probe resulting in ligated the first and the second probe and inclusion of the universal sequence and/or third barcode sequence into the bridge complementary strand. The bridge oligo or bridge oligo complex is extended from the 5' site or the 3' site complimentary to the ligated probes such that the target sequence identifier sequence present in the first probe or second probe is integrated into the bridge oligo or bridge oligo complex. Preferably, a polymerase is used that does not break up double stranded DNA, such as for instance a Taq polymerase, in order not to interfere with the ligation of the first to the second probe when both are annealed to the target sequence. In one embodiment, the bridge oligo, or one or more oligonucleotides of the plurality of bridge oligonucleotides, comprises, in a region not complementary to the first probe or the second probe, a plurality of universal base analogues to permit the incorporation of random sequences suitable for use as molecular barcode for target enumeration. These random sequences may thus become the third barcode. In such an embodiment, as part of step (v), a gap filling step is performed using polymerase and nucleotides in order to generate such random sequences.. In embodiment, plurality of universal base analogues is a plurality of 5-nitroindoles or deoxyinosines.

Before or after step (v), the ligated ligation complexes are optionally pooled from one or more target samples.

Subsequently, the ligated ligation complexes are allowed to dissociate from the target nucleotide sequence (step (vi)) and a target-specific probe is added and allowed to anneal to the ligated ligation complexes thereby forming amplification templates (step (vii)). The target-specific probe comprises a sequence corresponding to the target nucleotide sequence and thus specifically anneals to ligated ligation complexes that match with that sequence. The target-specific probe optionally includes a capture moiety such as biotin and/or nucleotide modifications (including but not limited to phosphorothioate, LNA and PNA modifications) which enhance its binding to the target or protect it from exonuclease activity. The inclusion of steps (vi) and (vii) allows selective amplification of variant sequences, for example a rare mutation, in a sample. As illustrated in Figure 3, if the target-specific probe sequence is chosen such that it matches with a variant sequence (e.g. rare mutation) to be detected, but does not match with non-variant sequences (e.g. bulk genome wild-type sequence), it will promote selective amplification of the variant sequence in the following steps, thus facilitation subsequent detection. Typically, in step (vii) a large surplus of the target-specific probe is added relative to the amount of target sequence, such that formation of amplification templates is favored over re-annealing of the ligated ligation complexes with the target sequence.

Next, nucleic acids are amplified from the amplification templates (step (viii)). As illustrated in Figure 3, amplification can initate from the bridge oligo and/or from the target-specific probe. Amplification from the annealed target-specific probe result in selective amplification of variant target sequences containing the target-specific probe sequence ("match") over non-variant target sequences not containg the target-specific probe sequence ("mismatch"). Amplification is performed using rolling circle amplification with a strand-displacing polymerase, such as phi29 polymerase (UniProtKB - P03680; DPOL_BPPH2) or a Bst polymerase (P52026; DPO1_GEOSE).

Single-stranded concatemeric sequences are obtained as a result of step (viii).

Optionally, provided a recognition sequence as specified in step (i) is present, a step (ix) is performed to obtain nucleic acid fragments by:
(a) cleaving the single-stranded concatemeric sequences obtained in step (viii), or
(b) subjecting the amplified one or more single-stranded concatemeric sequence obtained in step (viii) to annealing with a specific oligonucleotide containing a recognition sequence for an endonuclease wherein the oligonucleotide anneals with the recognition sequence specified in step (i) such that a recognition site for the endonuclease is obtained and cleaving the annealed complexes with said endonuclease.

Optionally, after amplification, the solid supports, if present, are removed and the supernatant is used for subsequent processing. For example, if the solid supports are magnetic particles, these may be removed using a magnet. In some other embodiments of the method of the invention, the interaction between the first capture moiety and the second capture moiety is disrupted immediately after step (v), after step (vi) or after step (vii). For example, if the first capture moiety is biotin and the second capture moiety is streptavidin, the interaction can be disrupted by adding excess soluble biotin. If the streptavidin is bound to magnetic particles, it can subsequently be removed using a magnet.

Next, in step (x), the concatemeric sequences obtained in step (viii) or, if step (ix) was performed, the nucleic acid fragments obtained in step (ix), are subjected to high-throughput sequencing technology to determine the barcode sequence(s).

Optionally, a PCR amplification is performed immediately prior to step (x) using primers which bind to universal parts of the first and second probes, wherein said primers optionally include adapter sequences for the subsequent sequencing in step (x).

In another embodiment, the sequencing in step (x) is performed using nanopore sequencing, wherein optionally the concatemeric sequence obtained in step (viii) is fragmented using transposition complexes. Suitable techniques for nanopore sequencing have been reviewed in Wang et al. (2021 Nat Biotechnol 39(11): 1348.

The identification of the presence and/or number of the target nucleotide sequence in the plurality of samples may be performed by determination of at least part of the first and/or second target specific portion, at least part of the first and/or second barcode, and/or at least part of the third barcode by high-throughput sequencing technology (steps (x) and (xi)), for example using a next-generation sequencing platform including without limiting, Illumina iSeq, MiSeq, HiSeq, NextSeq or NovaSeq. Preferably, the genetic target enumeration is permitted by counting the number of molecular barcodes per target and per sample. The samples are separated (de-convoluted) from the sequence data and the sequence targets quantified in silico after the DNA sequencing.

The advantages of the present invention include, but are not limited to quantification assay with low cost, high simplicity, high specificity, high sensitivity, high accuracy, high throughput, high scalability and high turn-over in comparison to traditional nucleic acid sequencing technologies. Another aspect of the present invention is that the methods of the present invention allow accurate and massively parallel quantification of plurality of nucleic acid targets in multiple samples including human and animal populations, and including large volumes of unpurified sample material. As mentioned, in a preferred embodiment, the sample, such as a urine sample, is used without any prior purification or concentration of nucleic acid. In another embodiment, the sample may be pre-treated, for instance lysing cells to expose nucleic acid. One particular advantage of the invention is to enable the detection and amplification of target sequence of interest using unique probe designs, i.e., probe triplet. The probes are designed with specially situated modified nucleotides that improve annealing and binding efficiency. Improvement in binding properties leads to higher assay specificity, sensitivity and accuracy. The methods of the present invention are likewise applicable for studying genetic variants and find application in diagnosis and prognosis, including but not limited to genotype the sample(s) for one or more sequences and/or polymorphisms, such as SNPs and/or indels, cancer diagnostics or fetal chromosomal disorders from maternal blood. In a preferred embodiment, for two or more samples or for two or more locus/allele combinations, barcode sequences are used to genotype the samples for one or more sequences and/or polymorphisms, such as SNPs and/or indels.

In another aspect, the invention provides a kit of parts comprising a plurality of containers, wherein at least one container comprises one or more sets of first probe and second probe, and at least one container comprises one or more bridge oligos or plurality of oligonucleotides capable of forming a bridge oligo complex,
wherein the first probe comprises, starting from the 5' end of the molecule, a first bridge oligo-specific sequence, optionally a first sequence barcode, and a first target specific portion at 3' end of first probe;
wherein the second probe comprises, starting from the 5' end of the molecule, a second target specific portion, optionally a second sequence barcode, and a second bridge oligo-specific sequence at 3' end of second probe;
wherein the bridge oligo or bridge oligo complex comprises sequences complementary to the first and second bridge oligo-specific sequences in the first and second probe, respectively, and optionally a third barcode; wherein at least one of the first sequence barcode or the second sequence barcode or the third barcode is present in the first probe or the second probe or the bridge oligo or bridge oligo complex, respectively;
wherein the kit further comprises a target-specific probe comprising a sequence corresponding to the target nucleotide sequence, wherein said target-specific probe is capable of annealing with the ligated ligation complexex;
and wherein optionally at least one of the first probe or the second probe or bridge oligo or bridge oligo complex comprises a recognition sequence for an endonuclease;
and wherein optionally the kit of parts further comprises an oligonucleotide capable of annealing with said recognition sequence such that a recognition site for said endonuclease is obtained.

Preferably, the 3' end of the first probes or the 5' end of the second probes, or both, are modified to permit chemical ligation of the first probes to the second probes.

Preferably, the bridge oligo or an oligonucleotide of the plurality of bridge oligonucleotides comprises one or more chemically modified nucleotides in the sequence complementary to a sequence of the first probe or in the sequence complementary to a sequence of the second probe, or both.

Preferably, the 3' end of the first probe or the 5' end of the second probe, or both, are modified to permit chemical ligation of the first probe to the second probe.

Preferably, the bridging portion of the first probe or the second probe, or both, or the bridge oligo or an oligonucleotide of the plurality of bridge oligonucleotides comprise(s) chemically modified bases to permit improved binding to the bridge oligo or bridge oligo complex.

In one particular embodiment, at least one container comprising the set of first and second probe and at least one container comprising the bridge oligo or a plurality of oligonucleotides capable of annealing to each other to form a bridge oligo complex, are one and the same container. In such case, the probes may be pre-annealed and have formed a ligated complex.

One particular advantage of the invention is to enable the detection and amplification of target sequence of interest using unique probe designs. The probes are designed with improved binding properties lead to higher assay specificity, sensitivity and accuracy. The present invention finds application in the area of molecular biology, evolutionary biology, metagenomics, genotyping and more specifically, but not limited to cancer diagnostics or fetal chromosomal disorders, including but not limited to genotype sample(s) for one or more sequences and/or polymorphisms, such as SNPs and/or indels.

In one particular preferred embodiment, the bridge oligo or bridge oligo complex comprises information to identify the sample and includes a unique barcode. In such case, the first and second probe is universally applicable to all samples (and only comprises information to identify the target). In one preferred embodiment, therefore, a method or a kit according to the invention is provided, wherein the bridge oligo or bridge oligo complex comprises a barcode that comprises a unique sequence that enables enumeration of the target sequences of each sample.

### EXAMPLES

### Method

### 1. Formation of probe complexes

Probe complexes contain sequences required for genomic targeting, sample indexing and constructing Illumina sequencing libraries.

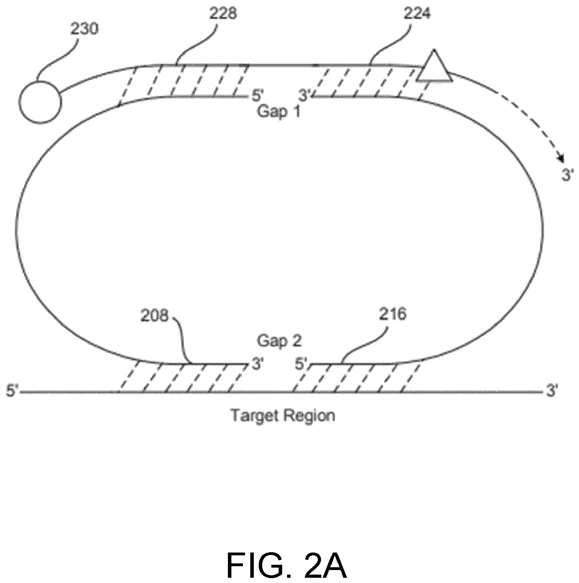

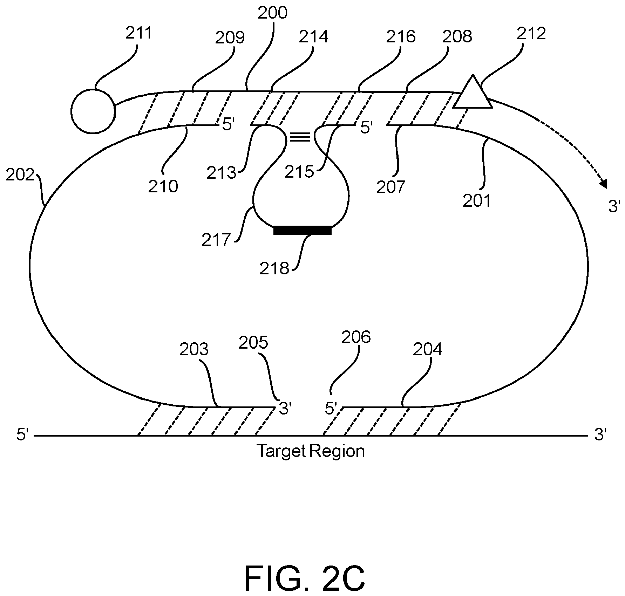

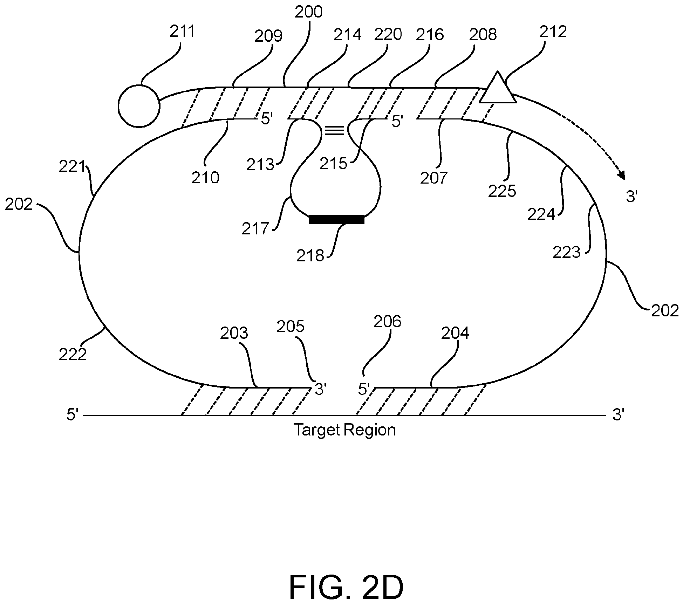

Three-part probe complexes are allowed to form (as illustrated in Figure 2), comprising:
(a) a first probe having, starting from the 5' end of the molecule, a first bridge oligo-specific sequence, and a first target specific portion at the 3' end of first probe;
(b) a second probe having, starting from the 5' end of the molecule, a second target specific portion, a second sequence barcode, and a second bridge oligo-specific sequence at the 3' end of second probe;
and (c) a bridge oligo having sequences complementary to the first bridge oligo-specific sequence and the second bridge oligo-specific sequence in the first probe and the second probe, respectively,

Probe complexes are built by combining all three parts (bridge, first arm and second arm) in equimolar amounts in annealing reaction. The reaction is carried out in a thermo cycler (Annealing program in Table 1).

**Table 1.**

| **Step** | | **Temperature** | **Time** |
|---|---|---|---|
| **1** | | +95°C | 5 min |
| **2** | | +95°C | 1 min |

| | -1°C/1 min, go to 2 40x | | |
|---|---|---|---|
| **3** | | +55°C | 10 min |
| **4** | | +55°C | 1 min |

| | -1°C/1 min, go to 4 35x | | |
|---|---|---|---|
| **5** | | +4°C | hold |

### 2. Target capture

Specific genomic regions containing the mutation(s) of interest are targeted. Purified DNA (for example from tissue, plasma, urine or saliva) can be used as sample or the samples can be non-purified, but only preprocessed, for example by boiling and/or centrifugation.

The probe complexes are hybridized to target regions via base sequence complementary interactions. To initiate the target capture, reaction probes and target DNA are mixed and incubated in a thermal cycler (Target capture and GapFill program in Table 2).

**Table 2.**

| **Step** | **Temperature** | **Time** | **Process** |
|---|---|---|---|
| 1 | +85°C | 4 min | denaturation |
| 2 | +75°C | 2,5 min | |
| 3 | +65°C | 2,5 min | |
| 4 | +55°C | 120 min | target capture |
| 5 | +50°C | 10 min | GapFlII |
| 6 | +45°C | 45 min | |
| 7 | +4°C | hold | |

### 3. GapFill reaction

After target capture, probe complexes are extended and ligated by adding a combination of Phusion DNA polymerase, nucleotides and Ampligase DNA ligase and incubating 45 minutes at +45°C.

### 4. Exonuclease treatments

After GapFill linear molecules are removed by adding 1µl of Thermolabile Exonuclease 1 (NEB, #M0568L) and 1µl of RecJF Exonuclease (NEB, #M0264L) and incubating 30 minutes in +37°C. Exonucleases are inactivated by incubating 12 minutes in +92°C.

### 5. Rolling Circle Amplification

After extension and ligation, circular probe molecules are aligned with the target-specific probes and subjected to Rolling Circle Amplification (RCA). For RCA reaction target capture reaction is mixed with boost oligoes and briefly denatured. The reaction is subsequently mixed with RCA reaction mix containing EquipPhi29 (Thermo Scientific) polymerase. Reaction is incubated at +42°C for 30 min - 2 hours. After RCA reaction the efficiency of reaction is analyzed by measuring the concentration of single stranded DNA (ssDNA) with Qubit fluorometer.

### 6. Enzymatic digestion

RCA reaction produces a long concatemeric ssDNA molecule having multiple copies of target library. Each complete target library is separated by EcoRI restriction enzyme recognition sequence. This sequence permits sequence-specific cutting of the long concatemer via annealing with a specific oligonucleotide containing the EcoRI restriction enzyme recognition sequence and release of ready target libraries. These libraries are ready for further analysis after a simple purification step. RCA products are digested with EcoRI for 1 hour at +37°C.

### 7. Library PCR

The digested RCA products are extended into sequencing libraries in a PCR reaction where the truncated sequencing adapters present in the right probe are extended into flow-cell compatible full-length sequencing adapters.

### 8. Library purification

After library PCR the library molecules are purified by extracting them from agarose gels after electrophoresis or with size selection beads (such as Macherey Nagel NucleoMag).

### 9. Sequencing

Purified MiSeq- or iSeq100-compatible libraries are subjected to sequence analysis using state of the art sequencing instruments. Importantly, the libraries can be converted to fit in any available sequencing platform by simple oligonucleotide modifications. Sequencing data is processed using a combination of Unix command line tools and Python and R programming languages. Briefly, the rationale for the sequence processing is to identify the probe sequences within each read, sequence the genomic area between them, and count the number of molecular barcodes associated with each genetic target.

### Experiment 1.

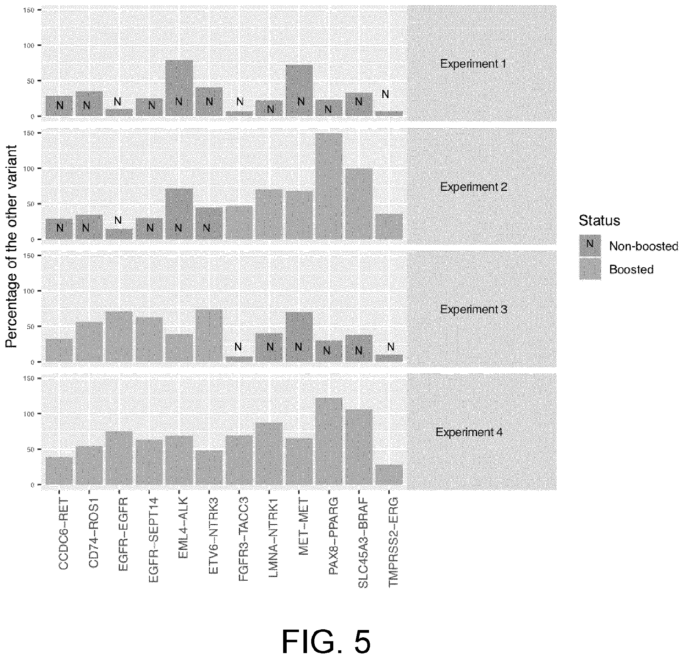

In the first experiment the probe mix was a collection of four differentially indexed probes resulting in four replicate reactions. They targeted twelve gene fusions listed in Figure 5. Target oligonucleotides had unique recognition sequences allowing identification of each target.

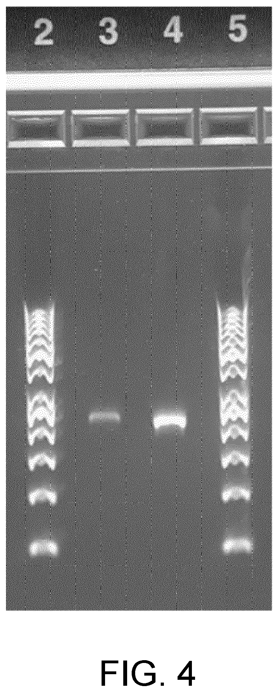

As a sample, two types of synthetic target oligonucleotides were mixed for each of the twelve gene fusions in equal concentrations. Target capture, extension and ligation reactions, rolling circle amplification and subsequent digestion with EcoRI were carried out as described above. An example of the resulting sequencing library is shown in Figure 4.

Ready libraries were sequenced with iSeq100 instrument and target regions were detected within the sequence data by matching the probe sequences within each read, identifying the genomic sequence area between the probe sequences and counting the molecular barcodes. The count data accurately reflected the boosting status of the respective gene fusion targets (Figure 5). In Fig 5 the data related to non-boosted is marked with letter "N" in the graph. Bars with no marking are related to boosted.

### DETAILED DESCRIPTION OF FIGURES 1, 2 AND 3

FIG 1 illustrates the workflow of one embodiment of the described invention. In step 1, nucleic acids (DNA or RNA) within a sample (102) are brought into contact with a set of ligation complexes (104). The ligation complexes anneal on the target nucleic acids (106). In step 2, the target-bound ligation complexes are optionally captured from the sample material, leaving behind sample impurities (103). In step 3, the annealed ligation complexes are ligated, resulting in ligated ligation complexes. In step 4, ligated ligation complexes from multiple samples (110) are pooled together (112). In step 5, the ligated ligation complexes are allowed to dissociate from the target nucleotide sequence, a target-specific probe comprising a sequence corresponding to the target nucleotide sequence is added and the target-specific probe anneals to the ligated ligation complexes. The target-specific probes anneal specifically to the rare mutations of choice (114) and optionally contain modifications (including but not limited to phosphorothioate modifications) which enhance their binding to the target or protect them from exonuclease activity. In step 6, the probe sequences are amplified by rolling circle amplification using phi29 polymerase or other strand displacing polymerase, resulting in long concatemeric copies of the probes. The amplification of the rare mutations is boosted with additional efficiency (116). In step 7, the concatemeric probe copies are optionally cleaved into monomeric units using a restriction endonuclease such as EcoRI or a homing nuclease such as I-CeuI and are optionally further amplified using PCR or emulsion PCR (117). In step 8, the amplified DNA is sequenced using next-generation DNA sequencing. In step 9, the DNA sequencing results are converted into target counts using a bioinformatic pipeline.

FIG. 2A illustrates gap filling between the first probe and the second probe according to an embodiment herein. Here, the bridge oligo contains Gap1 between the bridge sequence 1 (228), and bridge sequence 2 (224). Gap2 is formed between the target binding parts of probes 1 and 2 (208 and 216). These gaps are filled by introducing a polymerase and one or more nucleotides. For this process a mixture of Stoffel fragment, Taq polymerase or Phusion polymerase, and DNA ligase such as Ampligase can be used. The polymerase adds nucleotides (a) complimentary to the universal bridge oligo sequence and (b) complimentary to the target sequence and thereby fills in the two gaps i.e. gap 1 and gap 2 between the first probe and the second probe, and the subsequent action of the DNA ligase results in ligation of the first probe and the second probe complementary to the bridge oligo and the target sequence, into a circular complex.

FIG. 2B illustrates a principle structure of a probe quintet having a plurality of probe entities according to an embodiment herein. The plurality of probe entities include a first probe, a second probe and an bridge consisting of three oligos. Here the probe complex contains gaps between the first probe and the second bridge (228 and 236), between the second bridge and the second probe (240 and 222), between the first and third bridge oligos (238 and 242) and between the first and second probes (208 and 216). These gaps are filled by introducing a polymerase and one or more nucleotides. For this process a mixture of Stoffel fragment, Taq polymerase or Phusion polymerase, and DNA ligase such as Ampligase can be used. The polymerase fills these gaps and the subsequent action of the DNA ligase results in ligation of the probe and bridge oligos into a circular complex.

15-25 bases of the first probe includes an bridge binding sequence 1 (228), that optionally includes chemically modified bases for efficient bridge oligo binding referred as bridge sequence 1. The first probe further optionally includes following 10-20 bases from the 5' end including universal sequence used for library indexing (204) The first probe further optionally includes following 10-20 bases from the 5' end including segments of random nucleotides which form the molecule-specific barcode or sample-specific barcode referred to as barcode 1 (206). The first probe further includes following 15-30 bases from the 5' end, binds to the genetic target (208). Some or all of the nucleotides of 228 may include chemical modifications that increase the affinity of the probes to the target or the bridge (226). The last base of the first probe optionally includes a phosphate moiety for enzymatic ligation or a modification that permits chemical ligation to the 5' end of the adjacent probe referred to as modification 1 (210).

The first base of the second probe optionally includes a phosphate moiety for enzymatic ligation or a modification that permits chemical ligation to the 5' end of the adjacent probe referred to as modification 2 (214). The 15-30 bases from the 5' end of the second probe include a part of the second probe that binds to the genetic target (216). The following 10-20 bases from the 5' end of the second probe optionally include segments of random nucleotides which form the molecule-specific barcode or sample-specific barcode referred to as barcode 2 (218). The following 10-20 bases from the 5' end of the second probe optionally include a universal sequence (220). The last 15-25 bases of the second probe, referred as bridge sequence 8 (222), are reverse complementary to the bridge sequence 7 of the third bridge oligo (224). Some or all of the nucleotides of 208, 216, 222 or 228 may include chemical modifications that increase the affinity of the probes to the target or the bridge oligo.

The first 15-25 bases from the 5' end of the first bridge oligo, referred as bridge sequence 3 (226), are reverse complementary to the bridge sequence 1 of the second probe (228), and optionally include chemically modified nucleotides for increased binding. The last 15-25 bases of the first bridge oligo, referred as bridge sequence 2 (238), are reverse complementary to the bridge sequence 4 sequence of the second bridge oligo (236), and optionally include chemically modified nucleotides for increased binding. The 5' end of the first bridge oligo optionally includes a capture moiety (230) used for capturing the ligation complexes.

The first 15-25 bases from the 5' end of the second bridge oligo, referred as bridge sequence 5 (240) are reverse complementary to the bridge sequence 6 (242) of the third bridge oligo, and optionally include chemically modified nucleotides for increased binding. The last 15-25 bases of the second bridge oligo, referred as bridge sequence 4 (236), are reverse complementary to the bridge sequence 2 sequence of the first bridge oligo (238), and optionally include chemically modified nucleotides for increased binding.

The first 15-25 bases of the third bridge oligo from the 5' end, referred as bridge sequence 6 (242), are reverse complementary to the bridge sequence 5 sequence of the second bridge oligo (240), and optionally include chemically modified nucleotides for increased binding. The last 15-25 bases of the first bridge oligo, referred as bridge sequence 7 (224), are reverse complementary to the bridge sequence 8 sequence of the second probe (222), and optionally include chemically modified nucleotides for increased binding. The 3' end of the third bridge oligo optionally includes a phosphate (or other cleavable) moiety (234) to prevent extension during gap fill.

FIG. 2C illustrates an embodiment including the use of a barcode loop oligo. Here, the plurality of probe entities includes a first probe (202), a second probe (201), a bridge oligo (200) and a barcode loop oligo (217). Here the probe complex contains gaps between the first probe and the barcode loop oligo (210 and 213), between the second probe and the and the barcode loop oligo (207 and 215) and between the first and second probes (203 and 204). These gaps are filled by introducing a polymerase and one or more nucleotides. For this process, a mixture of Stoffel fragment, Taq polymerase or Phusion polymerase, and DNA ligase such as Ampligase can be used. The polymerase fills these gaps and the subsequent action of the DNA ligase results in ligation of the probe, bridge and barcode loop oligos into a circular complex.

15-25 bases of the first probe includes an bridge binding sequence (210), that optionally includes chemically modified bases for efficient bridge oligo binding. The first probe further includes following 15-30 bases from the 5' end, binds to the genetic target (203). Some or all of the nucleotides of 210 may include chemical modifications that increase the affinity of the probes to the target or the bridge (209). The last base of the frst probe optionally includes a phosphate moiety for enzymatic ligation or a modification that permits chemical ligation to the 5' end of the adjacent probe (205).

The first base of the second probe optionally includes a phosphate moiety for enzymatic ligation or a modification that permits chemical ligation to the 5' end of the adjacent probe (206). The 15-30 bases from the 5' end of the second probe include a part of the second probe that binds to the genetic target (204). The last 15-25 bases of the second probe (207), are reverse complementary to the bridge oligo (208). Some or all of the nucleotides of 203, 204, 207 or 210 may include chemical modifications that increase the affinity of the probes to the target or the bridge oligo.

The first 15-25 bases from the 5' end of the bridge oligo (209), are reverse complementary to the bridge-oligo specific sequence of the first probe (210), and optionally include chemically modified nucleotides for increased binding. The last 15-25 bases of the bridge oligo (208), are reverse complementary to the sequence of the second probe (207), and optionally include chemically modified nucleotides for increased binding. The 5' end of the bridge oligo optionally includes a capture moiety (211) used for capturing the ligation complexes. Furthermore, the bridge oligo comprises sequences 214 and 216, complementary to sequences 213 and 215 of the barcode loop oligo. The 3' end of the bridge oligo optionally includes a phosphate (or other cleavable) moiety (212) to prevent extension during gap fill.

The first 15-25 bases from the 5' end of the barcode loop oligo (215) are reverse complementary to bridge oligo sequence 216. The barcode loop oligo comprises a loop region comprising a barcode (218). The last 15-25 bases of the barcode loop oligo (213) are reverse complementary to bridge oligo sequence 214.

FIG. 2D illustrates a principle structure of a probe quadruplet according to an embodiment herein. The plurality of probe entities includes a first probe (202), a second probe (201), a bridge oligo (200) and a barcode loop oligo (217). Here the probe complex contains gaps between the first probe and the barcode loop oligo (210 and 213), between the second probe and the and the barcode loop oligo (207 and 215) and between the first and second probes (203 and 204). These gaps are filled by introducing a polymerase and one or more nucleotides. For this process, a mixture of Stoffel fragment, Taq polymerase or Phusion polymerase, and DNA ligase such as Ampligase can be used. The polymerase fills these gaps and the subsequent action of the DNA ligase results in ligation of the probe, bridge and barcode loop oligos into a circular complex.

15-25 bases of the first probe includes an bridge binding sequence (210), that optionally includes chemically modified bases for efficient bridge oligo binding. The first probe further includes a binding site for an amplification primer (221) and a barcode sequence (222), and 15-30 bases from the 5' end, a sequence binding to the genetic target (203). Some or all of the nucleotides of 210 may include chemical modifications that increase the affinity of the probes to the target or the bridge (209). The last base of the first probe optionally includes a phosphate moiety for enzymatic ligation or a modification that permits chemical ligation to the 5' end of the adjacent probe (205).

The first base of the second probe optionally includes a phosphate moiety for enzymatic ligation or a modification that permits chemical ligation to the 5' end of the adjacent probe (206). The 15-30 bases from the 5' end of the second probe include a part of the second probe that binds to the genetic target (204). The second probe further includes a binding site for an amplification primer (223), a sequencing adapter sequence (224), a recognition site for a restriction endonuclease such as EcoRI (225) and another sequencing adapter sequence (207). The last 15-25 bases of the second probe (207), are reverse complementary to the bridge oligo (208). Some or all of the nucleotides of 203, 204, 207 or 210 may include chemical modifications that increase the affinity of the probes to the target or the bridge oligo.

The first 15-25 bases from the 5' end of the bridge oligo (209), are reverse complementary to the bridge-oligo specific sequence of the first probe (210), and optionally include chemically modified nucleotides for increased binding. The last 15-25 bases of the bridge oligo (208), are reverse complementary to the sequence of the second probe (207), and optionally include chemically modified nucleotides for increased binding. The part of the bridge oligo not reverse-complementary with either end of the barcode loop oligo (220) optionally contains a recognition site for a restriction endonuclease. The 5' end of the bridge oligo optionally includes a capture moiety (211) used for capturing the ligation complexes. Furthermore, the bridge oligo comprises sequences 214 and 216, complementary to sequences 213 and 215 of the barcode loop oligo. The 3' end of the bridge oligo optionally includes a phosphate (or other cleavable) moiety (212) to prevent extension during gap fill.

The first 15-25 bases from the 5' end of the barcode loop oligo (215) are reverse complementary to bridge oligo sequence 216. The barcode loop oligo comprises a loop region comprising a barcode (218). The last 15-25 bases of the barcode loop oligo (213) are reverse complementary to bridge oligo sequence 214.

FIG. 3 illustrates how the workflow of one embodiment of the described invention enhances the detection of rare mutations in samples also containing bulk genome not containing the mutation. In step 1, target-bound ligation complexes are subjected to gap filling and ligation. In step 2, the ligated ligation complexes are allowed to dissociate from the target nucleotide sequence, a target-specific probe comprising a sequence corresponding to the target nucleotide sequence is added and the target-specific probe anneals to the ligated ligation complexes. In step 3 and 4, the probe sequences are amplified by rolling circle amplification using phi29 polymerase or other strand displacing polymerase, resulting in long concatemeric copies of the probes. Amplification starting from the target-specific probe only occurs if the target-specific prone matches with the target sequence. If there is a mismatch, there will only be amplification starting from the bridge oligo.

## Claims

1. A method for the detection of one or more target nucleotide sequence in a sample, the method comprising the steps of:
(i) providing for each target nucleotide sequence in the sample:
a first probe, a second probe and a bridge oligo or a plurality of oligonucleotides capable of annealing to each other to form a bridge oligo complex,
wherein the first probe comprises, starting from the 5' end of the molecule, a first bridge oligo-specific sequence, optionally a first sequence barcode, and a first target specific portion at the 3' end of first probe;
wherein the second probe comprises, starting from the 5' end of the molecule, a second target specific portion, optionally a second sequence barcode, and a second bridge oligo-specific sequence at the 3' end of second probe;
wherein the bridge oligo or bridge oligo complex contains sequences complementary to the first bridge oligo-specific sequence and the second bridge oligo-specific sequence in the first probe and the second probe, respectively, and optionally a third barcode;
wherein at least one of the first sequence barcode or the second sequence barcode or the third barcode is present in the first probe or the second probe or the bridge oligo or bridge oligo complex, respectively;
and wherein optionally at least one of the first probe or the second probe or the bridge oligo or bridge oligo complex comprises a recognition sequence for an endonuclease;
(ii) contacting, for each of the one or more target nucleotide sequence, the first probe and the second probe with the bridge oligo or plurality of oligonucleotides capable of annealing to each other to form a bridge oligo complex and allow self-annealing into a plurality of ligation complexes;
(iii) contacting nucleic acids present in the sample to be tested for the target nucleotide sequences with the ligation complexes;
(iv) allowing the first target specific portion and the second target specific portion of the respective first probe and the second probe to hybridize to essentially adjacent sections on the target sequence, thereby forming a hybridization complex;
(v) ligating the probes in the hybridization complexes to provide ligated ligation complexes;
(vi) allowing the ligated ligation complexes to dissociate from the target nucleotide sequence;
(vii) adding a target-specific probe comprising a sequence corresponding to the target nucleotide sequence, wherein said target-specific probe is capable of annealing with the ligated ligation complexes, and allowing the target-specific probe to anneal to the ligated ligation complexes thereby forming amplification templates;
(viii) amplifying nucleic acids from the amplification templates using rolling circle amplification with a strand-displacing polymerase thereby obtaining single-stranded concatemeric sequences;
(ix) optionally, provided a recognition sequence as specified in step (i) is present, performing a step to obtain nucleic acid fragments by:
(a) cleaving the single-stranded concatemeric sequences obtained in step (viii), or
(b) subjecting the amplified one or more single-stranded concatemeric sequence obtained in step (viii) to annealing with a specific oligonucleotide containing a recognition sequence for an endonuclease wherein the oligonucleotide anneals with the recognition sequence specified in step (i) such that a recognition site for the endonuclease is obtained and cleaving the annealed complexes with said endonuclease;
(x) subjecting the concatemeric sequence obtained in step (viii) or the nucleic acid fragments obtained in step (ix) to sequencing technology to determine the barcode sequence(s); and
(xi) identifying the presence and/or number of the target nucleotide sequence in the sample by determination of at least part of the first target specific portion and/or the second target specific portion, and/or at least part of the first barcode and/or the second barcode, and/or at least part of the third barcode.

2. Method according to claim 1, wherein method is for the high-throughput detection of one or more target nucleotide sequence in a plurality of samples, wherein a plurality of samples is provided and wherein, preferably, step (ii) is performed for each of the samples in a separate tube.

3. Method according to claim 2, wherein a plurality of the samples is pooled prior to step (viii).

4. Method according to any one of the preceding claims, wherein at least one of: the first probe, the second probe, the bridge oligo, or an oligonucleotide of the plurality of bridge oligonucleotides, comprises a first capture moiety, and wherein between steps (iv) and (v) an intermediate step (iv)(a) is performed which comprises bringing the hybridization complex in contact with a solid support comprising a second capture moiety, allowing the first capture moiety and the second capture moiety to interact such that the hybridization complexes become linked to the solid support and separating the solid-support-linked hybridization complexes from components of the samples that are not linked to the solid-support.

5. Method according to claim 4, wherein the method does not comprise a step of enriching for nucleic acids prior to step (iv)(a).

6. Method according to claims 4 or 5, wherein the first capture moiety is a biotin moiety and the second capture moiety is a streptavidin moiety or an avidin moiety.

7. Method according to any one of the preceding claims, wherein the sample or the plurality of samples includes a blood sample, a saliva sample, a urine sample or a feces sample.

8. Method according to any one of the preceding claims,
wherein the bridge oligo, or one or more oligonucleotides of the plurality of bridge oligonucleotides, comprises, in a region not complementary to the first probe or the second probe, a plurality of universal base analogues to permit the incorporation of random sequences suitable for use as molecular barcode for target enumeration, and
wherein, prior to step (v), a gap filling step is performed using polymerase and nucleotides in order to generate such random sequences.

9. Method according to claim 8, wherein said plurality of universal base analogues is a plurality of 5-nitroindoles.

10. Method according to any one of the preceding claims, wherein the method comprises the use of a plurality of oligonucleotides capable of annealing to each other to form a bridge oligo complex and wherein said plurality of oligonucleotides comprises a barcode loop oligo,
wherein the barcode loop oligo comprises, starting from the 5' end of the molecule, a third bridge oligo-specific sequence, a barcoded loop sequence, which may optionally comprise the third barcode, and a fourth bridge oligo-specific sequence, and
wherein the one or more other bridge oligo comprises sequences complementary to the third bridge oligo-specific sequence and the fourth bridge oligo-specific sequence in the barcode loop oligo.

11. Method according to any one of the preceding claims, wherein the bridge oligo or bridge oligo complex comprises:
(i) one to five 3' protruding bases, and/or
(ii) 3' phosphate, and/or
(iii) one or more phosphorothioate modifications within three positions from the 3' end.

12. Method according to any one of the preceding claims, wherein the 3' end of the first probe or the 5' end of the second probe, or both, are modified to permit chemical ligation of the first probe to the second probe.

13. Method according to any one of the preceding claims, wherein the bridging portion of the first probe or the second probe, or both, or the bridge oligo or an oligonucleotide of the plurality of bridge oligonucleotides comprise(s) chemically modified bases to permit improved binding to the bridge oligo or bridge oligo complex.

14. Method according to any one of the preceding claims, wherein the first target specific portion, the second target specific portion, the first bridge oligo-specific sequences, and/or the second bridge oligo-specific sequences, contain independently from one another, one or more chemically modified nucleotide.

15. Method according to any one of the preceding claims, wherein the bridge oligo or an oligonucleotide of the plurality of bridge oligonucleotides, comprises one or more chemically modified nucleotides.

16. Method according to any one of the preceding claims, wherein step (viii) is performed using a phi29 polymerase or a Bst polymerase.

17. Method according to any one of claim the preceding claims, wherein a PCR amplification is performed immediately prior to step (x) using primers which bind to universal parts of the first and second probes, wherein said primers optionally include adapters for subsequent sequencing in step (x).

18. Method according to any one of the preceding claims, wherein the sequencing in step (x) is performed using nanopore sequencing, wherein optionally the concatemeric sequence obtained in step (viii) is fragmented using transposition complexes.

19. Method according to any one of the preceding claims, wherein genetic target enumeration is permitted by counting the number of molecular barcodes per target and per sample.

20. Method according to any one of the preceding claims, wherein for two or more samples or for two or more locus/allele combinations, barcode sequences are used to genotype the sample(s) for one or more sequences and/or polymorphisms, such as SNPs and/or indels.

21. Kit of parts comprising a plurality of containers, wherein at least one container comprises one or more sets of first probe and second probe, and at least one container comprises one or more bridge oligos or plurality of oligonucleotides capable of forming a bridge oligo complex,
wherein the first probe comprises, starting from the 5' end of the molecule, a first bridge oligo-specific sequence, optionally a first sequence barcode, and a first target specific portion at 3' end of first probe;
wherein the second probe comprises, starting from the 5' end of the molecule, a second target specific portion, optionally a second sequence barcode, and a second bridge oligo-specific sequence at 3' end of second probe;
wherein the bridge oligo or bridge oligo complex comprises sequences complementary to the first and second bridge oligo-specific sequences in the first and second probe, respectively, and optionally a third barcode;
wherein at least one of the first sequence barcode or the second sequence barcode or the third barcode is present in the first probe or the second probe or the bridge oligo or bridge oligo complex, respectively;
wherein the kit further comprises a target-specific probe comprising a sequence corresponding to the target nucleotide sequence, wherein said target-specific probe is capable of annealing with the ligated ligation complexex;
and wherein optionally at least one of the first probe or the second probe or bridge oligo or bridge oligo complex comprises a recognition sequence for an endonuclease;
and wherein optionally the kit of parts further comprises an oligonucleotide capable of annealing with said recognition sequence such that a recognition site for said endonuclease is obtained.
